(19) Europäisches Patentamt
European Patent Office
Office européen des brevets

(11) Veröffentlichungsnummer: **0 039 520**
**B1**

(12) # EUROPÄISCHE PATENTSCHRIFT

(45) Veröffentlichungstag der Patentschrift: **11.04.84**

(51) Int. Cl.³: **C 07 D277/50**, C 07 D279/12, A 01 N 47/24, C 07 D417/12

(21) Anmeldenummer: **81103449.5**

(22) Anmeldetag: **06.05.81**

(54) **Carbamoyloxime, Verfahren und Mittel zu deren Herstellung, Schädlingsbekämpfungsmittel, enthaltend solche Verbindungen, sowie Verwendung solcher Verbindungen als Schädlingsbekämpfungsmittel.**

(30) Priorität: **07.05.80 CH 3555/80**
**10.04.81 CH 2407/81**

(43) Veröffentlichungstag der Anmeldung: **11.11.81 Patentblatt 81/45**

(45) Bekanntmachung des Hinweises auf die Patenterteilung: **11.04.84 Patentblatt 84/15**

(84) Benannte Vertragsstaaten:
**AT BE CH DE FR GB IT LI NL**

(56) Entgegenhaltungen:
**EP-A- 0 011 221**
**DE-A- 2 222 464**
**US-A- 3 790 566**
**US-A- 3 894 150**
**US-A- 4 071 627**
**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft**
**CH-4002 Basel (CH)**

(72) Erfinder: **Lüthy, Christoph, Dr.**
**Zielackerstrasse 13**
**CH-8603 Schwerzenbach (CH)**
Erfinder: **Winternitz, Paul, Dr.**
**Am Pfisterhölzli 50**
**CH-8606 Greifensee (CH)**

(74) Vertreter: **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 039 520 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Carbamoyloxime, Verfahren und Mittel zu deren Herstellung, Schädlingsbekämpfungsmittel, enthaltend solche Verbindungen, sowie Verwendung solcher Verbindungen als Schädlingsbekämpfungsmittel

Die Erfindung betrifft Carbamoyloxime der allgemeinen Formel

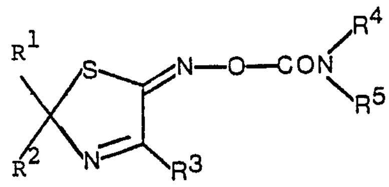

(I)

worin

$R^1$ und $R^2$ Wasserstoff, $C_{1-4}$-Alkyl oder $C_{3-6}$-Cycloalkyl, oder zusammen mit dem Kohlenstoffatom, an das sie geknüpft sind, einen 4- bis 6-gliedrigen, gesättigten Kohlenwasserstoffring,

$R^3$ Wasserstoff, $C_{1-4}$-Alkyl oder $C_{3-6}$-Cycloalkyl,

$R^4$ Wasserstoff und

$R^5$ $C_{1-4}$-Alkyl, $C_{2-5}$-Alkenyl oder $C_{3-6}$-Cycloalkyl, oder

$R^4$ $C_{1-4}$-Alkyl und

$R^5$ $C_{1-4}$-Alkyl, ($C_{1-4}$-Alkyl)carbonyl, Tri($C_{1-4}$-alkyl)silyl oder eine Gruppe (a)

$$—S_m—R^6 \qquad (a)$$

$R^6$ $C_{1-8}$-Alkyl ; $C_{1-4}$-Halogenalkyl ; Phenyl, gegebenenfalls mit Halogen, $C_{1-4}$-Alkyl und/oder Trifluormethyl substituiert ; oder eine Gruppe (b) oder (c)

$$—NR^7R^8 \qquad (b)$$

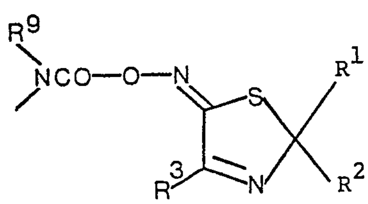

(c)

$R^7$ und $R^8$ $C_{1-4}$-Alkyl oder $C_{1-4}$-Halogenalkyl, oder zusammen mit dem Stickstoffatom, an das sie geknüpft sind, eine Piperidino- oder Morpholinogruppe,

$R^9$ $C_{1-4}$-Alkyl und

m 1 oder 2 bedeuten.

Die Verbindungen der Formel I sind Schädlingsbekämpfungsmittel und eignen sich insbesondere zur Bekämpfung von Insekten, Milben, Nematoden und Mollusken. Somit umfasst die Erfindung auch Schädlingsbekämpfungsmittel, die eine oder mehrere der Verbindungen der Formel I enthalten, Verfahren zur Herstellung dieser Verbindungen und Mittel sowie die Verwendung solcher Mittel.

Der Ausdruck « $C_{1-4}$-Alkyl » umfasst, wenn nicht ausdrücklich anderweitig definiert, sowohl geradkettige als auch verzweigte Alkylgruppen, wie Methyl, Aethyl, n-Propyl, Isopropyl, n-Butyl, Isobutyl und tert.-Butyl. Dies gilt auch für $C_{1-4}$-Alkylgruppe enthaltende Reste, wie $C_{1-4}$-Alkylcarbonyl und $C_{1-4}$-Halogenalkyl. Unter $C_{3-6}$-Cycloalkyl sind Cyclopropyl, Cyclobutyl, Cyclopentyl und Cyclohexyl zu verstehen. Der Ausdruck « $C_{2-5}$-Alkenyl » umfasst sowohl geradkettige als auch verzweigte Reste, wie Vinyl, Allyl, Butenyl oder Pentenyl. Der Ausdruck « Halogen » umfasst Fluor, Chlor, Brom und Jod. Wenn in einem substituierten Phenylrest zwei oder mehr Substituenten vorhanden sind, können die Substituenten gleich oder verschieden sein.

Die Erfindung betrifft auch die syn- und anti- Formen der Verbindungen der Formel I, wobei die syn(Z)-Form bevorzugt ist. Da asymmetrische Kohlenstoffatome vorhanden sein können, liegen in solchen Fällen optische Antipoden vor. Durch das Vorliegen der Doppelbindung in den Verbindungen der Formel I, worin $R^5$ $C_{2-5}$-Alkenyl bedeutet, tritt für diese Verbindungen zusätzlich geometrische Isomerie auf. Die Formel I soll demnach all diese möglichen isomeren Formen umfassen.

Hat $R^1$ oder $R^2$ die Bedeutung $C_{1-4}$-Alkyl, so ist dies vorzugsweise Methyl oder Aethyl, insbesondere Methyl. Darüber hinaus übersteigt die Gesamtzahl der Kohlenstoffatome in den Substituenten $R^1$ und $R^2$ vorzugsweise die Zahl 4 nicht.

Im Falle, dass $R^1$ und $R^2$ zusammen mit dem Kohlenstoffatom einen 4- bis 6-gliedrigen, gesättigten

Kohlenwasserstoffring bedeuten, so ist dieser Ring vorzugsweise ein 5- oder 6-gliedriger Ring.

$R^1$ und $R^2$ bedeuten vorzugsweise je Wasserstoff oder $C_{1-4}$-Alkyl.

Bedeutet $R^3$ $C_{1-4}$-Alkyl, so ist dies vorzugsweise Methyl.

Hat $R^4$ die Bedeutung $C_{1-4}$-Alkyl, so ist dies vorzugsweise Methyl.

$R^5$ in der Bedeutung $C_{1-4}$-Alkyl, $C_{2-5}$-Alkenyl, $C_{3-6}$-Cycloalkyl, ($C_{1-4}$-Alkyl)carbonyl und Tri ($C_{1-4}$-Alkyl)-silyl ist vorzugsweise Methyl, Allyl, Cyclopropyl, Acetyl bzw. Trimethylsilyl oder Tri (tert.-butyl)silyl.

Hat $R^6$ in der Gruppe (a) die Bedeutung $C_{1-4}$-Halogenalkyl, so ist dies vorzugsweise Perhalomethyl, insbesondere Trichlormethyl, Dichlorfluormethyl oder Trifluormethyl.

$R^5$ ist vorzugsweise $C_{1-4}$-Alkyl, insbesondere Methyl, wenn $R^4$ Wasserstoff bedeutet.

Eine ganz besonders bevorzugte Verbindung der Formel I ist 5-Oxo-2,2,4-trimethyl-3-thiazolin-O-(methylcarbamoyl)-oxim.

Weitere Vertreter von Verbindungen der Formel I sind :

5-Oxo-4-methyl-3-thiazolin-O-(methylcarbamoyl)oxim,
5-Oxo-2-Cyclopropyl-2,4-dimethyl-3-thiazolin-O-(methyl-carbamoyl)oxim,
5-Oxo-2,2,4-trimethyl-3-thiazolin-O-(allylcarbamoyl)-oxim,
5-Oxo-2,2,4-trimethyl-3-thiazolin-O-(cyclopropylcarbamoyl)oxim,
5-Oxo-2,2,4-trimethyl-3-thiazolin-O-(N-acetyl-N-methylcarbamoyl)oxim,
5-Oxo-2,2,4-trimethyl-3-thiazolin-O-(N-methyl-N-trimethylsilylcarbamoyl)oxim,
5-Oxo-2,2,4-trimethyl-3-thiazolin-O-(N-methyl-N-(methylsulfenyl)carbamoyl)oxim,
5-Oxo-2,2,4-trimethyl-3-thiazolin-O-(N-methyl-N-(methylthiosulfenyl)carbamoyl)oxim,
5-Oxo-2,2,4-trimethyl-3-thiazolin-O-(N-isopropylthiosulfenyl-N-methylcarbamoyl)oxim,
5-Oxo-2,2,4-trimethyl-3-thiazolin-O-(N-tert. butylthiosulfenyl-N-methylcarbamoyl)oxim,
5-Oxo-2,2,4-trimethyl-3-thiazolin-O-(N-methyl-N-(n-octylthiosulfenyl)carbamoyl)oxim,
5-Oxo-2,2,4-trimethyl-3-thiazolin-O-(N-trichlormethylsulfenyl-N-methylcarbamoyl)oxim,
5-Oxo-2,2,4-trimethyl-3-thiazolin-O-(N-dichlorfluormethylsulfenyl-N-methylcarbamoyl)oxim,
5-Oxo-2,2,4-trimethyl-3-thiazolin-O-(N-trifluormethylsulfenyl-N-methylcarbamoyl)oxim,
5-Oxo-2,2,4-trimethyl-3-thiazolin-O-(N-[(4-chlorphenyl)sulfenyl]-N-methylcarbamoyl)oxim,
5-Oxo-2,2,4-trimethyl-3-thiazolin-O-(N-methyl-N-[(2-methylphenyl)sulfenyl] carbamoyl)oxim,
5-Oxo-2,2,4-trimethyl-3-thiazolin-O-(N-methyl-N-[(4-methylphenyl)sulfenyl] carbamoyl)oxim,
5-Oxo-2,2,4-trimethyl-3-thiazolin-O-(N-[(3-4-dimethylphenyl)sulfenyl]-N-methylcarbamoyl)oxim,
5-Oxo-2,2,4-trimethyl-3-thiazolin-O-(N-methyl-N-[(3-trifluormethylphenyl)sulfenyl] carbamoyl)oxim,
5-Oxo-2,2,4-trimethyl-3-thiazolin-O-(N-[(4-tert.-butylphenyl)thiosulfenyl]-N-methylcarbamoyl)oxim,
5-Oxo-2,2,4-trimethyl-3-thiazolin-O-(N-dimethylaminosulfenyl-N-methylcarbamoyl)oxim,
5-Oxo-2,2,4-trimethyl-3-thiazolin-O-(N-di(2-chloräthyl)aminosulfenyl-N-methylcarbamoyl)oxim und
N,N'-Bis-(2,2,4-trimethyl-3-thiazolin-5-O-(methyl-carbamoyl)oximino)-disulfid.

Das erfindungsgemässe Verfahren zur Herstellung der Verbindungen der Formel I ist dadurch gekennzeichnet, dass man

a) ein Oxim der Formel

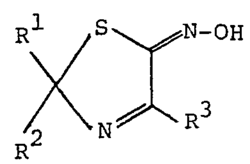

(II)

worin $R^1$, $R^2$ und $R^3$ die oben angegebenen Bedeutungen besitzen, mit einem Isocyanat der Formel

$$R^5—N = C = O \qquad \text{(III)}$$

worin $R^5$ $C_{1-4}$-Alkyl, $C_{2-5}$-Alkenyl oder $C_{3-6}$-Cycloalkyl bedeutet zweckmässigerweise in Gegenwart eines Verdünnungsmittels und gegebenenfalls in Gegenwart eines Katalysators umsetzt,

b) ein Oxim der Formel II, wie oben definiert, mit einem Carbamoylhalogenid der Formel

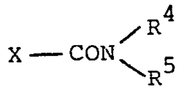

(IV)

worin $R^4$ und $R^5$ die oben angegebenen Bedeutungen besitzen und X ein Halogenatom bedeutet, zweckmässigerweise in Gegenwart eines Verdünnungsmittels und eines säurebindenden Mittels umsetzt,

c) ein Oxim der Formel II, wie oben definiert, mit Phosgen und anschliessend einem Amin der Formel

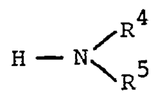 (V)

worin $R^4$ und $R^5$ die oben angegebenen Bedeutungen besitzen, zweckmässigerweise in Gegenwart eines Verdünnungsmittels und eines säurebindenden Mittels umsetzt, oder

d) ein gegebenenfalls nach einem der obigen Verfahrensvarianten erhaltenes Carbamoyloxim der Formel

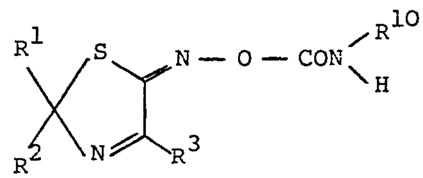 (I')

worin $R^1$, $R^2$ und $R^3$ die oben angegebenen Bedeutungen besitzen und $R^{10}$ $C_{1-4}$-Alkyl bedeutet, mit einem Sulfenylchlorid der Formel

$$Cl—S_m—R^{11} \quad (VI)$$

worin m 1 oder 2 und $R^{11}$ eine der oben angegebenen Bedeutungen von $R^6$ oder Chlor bedeuten, zweckmässigerweise in Gegenwart eines Verdünnungsmittels und eines säurebindenden Mittels umsetzt.

Die Verfahrensvariante a) führt zu Verbindungen der Formel I, worin $R^4$ Wasserstoff und $R^5$ $C_{1-4}$-Alkyl, $C_{2-5}$-Alkenyl oder $C_{3-6}$-Cycloalkyl bedeuten.

Für die Umsetzung gemäss Verfahrensvariante a) kommen als Verdünnungsmittel vorzugsweise inerte organische Lösungsmittel in Frage. Hierzu gehören vorzugsweise Aether und ätherartige Verbindungen, wie 1,2-Dimethoxyäthan, Tetrahydrofuran und Dioxan ; Ketone wie Diäthylketon, insbesondere Aceton und Methyläthylketon ; Nitrile wie Propionitril, insbesondere Acetonitril ; Formamide wie Dimethylformamid ; und halogenierte Kohlenwasserstoffe, wie Methylenchlorid, Tetrachlorkohlenstoff und Chloroform.

Bevorzugte Katalysatoren für diese Verfahrensvariante sind tertiäre Basen, wie Triäthylamin, Pyridin und 1,4-Diazabicyclo [2.2.2] octan ; und zinnorganische Verbindungen, wie Dibutylzinndiacetat.

Die Reaktionstemperaturen können bei der Durchführung dieser Verfahrensvariante in einem grossen Bereich variiert werden. Im allgemeinen wird zwischen 0 °C und 100 °C gearbeitet, vorzugsweise zwischen 20 °C und 50 °C. Bei der Anwesenheit eines Lösungsmittels wird zweckmässigerweise beim Siedepunkt des jeweiligen Lösungsmittels gearbeitet.

Bei der Durchführung dieser Verfahrensvariante arbeitet man vorzugsweise mit einem Ueberschuss an Isocyanat der Formel III. Zur Isolierung der so erhaltenen Verbindung der Formel I, worin $R^4$ Wasserstoff bedeutet, kann das gegebenenfalls vorhandene Lösungsmittel entfernt werden, z. B. durch Abdestillieren, und der Rückstand nach üblichen Methoden aufgearbeitet werden.

Für die Durchführung der Verfahrensvariante b) kommen als Verdünnungsmittel vorzugsweise inerte organische Lösungsmittel in Frage. Hierzu gehören vorzugsweise die bei der Verfahrensvariante a) aufgezählten Lösungsmittel.

Die Umsetzung nach Verfahrensvariante b) wird zweckmässigerweise in Gegenwart eines säurebindenden Mittels vorgenommen. Man kann alle üblicherweise verwendbaren anorganischen und organischen säurebindenden Mittel zugeben. Hierzu gehören vorzugsweise Natriumhydrid ; Alkalicarbonate, wie beispielsweise Natriumcarbonat, Kaliumcarbonat und Natriumhydrogencarbonat ; ferner niedere tertiäre Alkylamine, Cycloalkylamine oder Arylalkylamine, wie beispielsweise Triäthylamin und Dimethylbenzylcyclohexylamin ; weiterhin Pyridin und Diazabicyclooctan.

Die Reaktionstemperaturen können bei der Durchführung dieser Verfahrensvariante in einem grossen Bereich variiert werden. Im allgemeinen arbeitet man zwischen 0 °C und 100 °C, vorzugsweise zwischen 0 °C und 40 °C.

Bei der Durchführung dieser Verfahrensvariante setzt man vorzugsweise auf 1 Mol der Verbindung der Formel II 1 bis 2 Mol an Carbamoylhalogenid der Formel IV ein. Es hat sich als vorteilhaft erwiesen, das säurebindende Mittel in geringem Ueberschuss (bis ca. 30 Gewichtsprozent) einzusetzen. Die Isolierung der so erhaltenen Verbindung der Formel I erfolgt in üblicher Weise.

Für die Umsetzung gemäss Verfahrensvariante c) kommen als Verdünnungsmittel vorzugsweise inerte organische Lösungsmittel in Frage, insbesondere Aether und ätherartige Verbindungen, wie 1,2-Dimethoxyäthan, Tetrahydrofuran und Dioxan ; Nitrile wie Acetonitril ; Formamide wie Dimethylformamid ; und Kohlenwasserstoffe wie Toluol.

Die Umsetzung nach Verfahrensvariante c) wird zweckmässigerweise in Gegenwart eines säurebindenden Mittels vorgenommen. Man kann alle üblicherweise verwendbaren anorganischen und organischen säurebindenden Mittel zugeben, zu denen vorzugsweise die im Zusammenhang mit der Verfahrensvariante b) oben erwähnten gehören.

Die Reaktionstemperaturen können bei der Durchführung dieser Verfahrensvariante in einem grossen Bereich variiert werden. Im allgemeinen liegen sie zweckmässigerweise zwischen 0 °C und 100 °C, vorzugsweise zwischen 0 °C und 40 °C.

Zweckmässigerweise wird das Verfahren als ein Eintopfverfahren durchgeführt. Man setzt erst Phosgen mit dem Oxim der Formel II um, und dann gibt man zum Reaktionsgemisch das Amin der Formel V zu. Es hat sich als vorteilhaft erwiesen, auf 1 Mol des Oxims der Formel II 1 bis 1,5 Mol Phosgen und 1 bis 1,5 Mol des Amins der Formel V einzusetzen. Ferner wird das säurebindende Mittel vorzugsweise in geringem Ueberschuss, z. B. bis ca. 30 Gewichtsprozent, verwendet. Die Isolierung der so erhaltenen Verbindung der Formel I erfolgt in üblicher Weise.

Die Verfahrensvariante d) führt zu Verbindungen der Formel I, worin $R^4$ $C_{1\text{-}4}$-Alkyl und $R^5$ eine Gruppe (a) bedeuten.

Bei der Durchführung der Verfahrensvariante d) kommen als Verdünnungsmittel und säurebindende Mittel vorzugsweise diejenigen in Frage, die im Zusammenhang mit der Verfahrensvariante a) bzw. b) oben erwähnt werden. Die Reaktionstemperaturen liegen im allgemeinen zweckmässigerweise zwischen 0 °C und 100 °C, vorzugsweise zwischen 0 °C und 50 °C.

Falls ein Sulfenylchlorid der Formel VI, worin $R^{11}$ Chlor bedeutet, in dieser Verfahrensvariante verwendet wird, werden die Ausgangsmaterialien vorzugsweise in etwa stöchiometrischen Mengen eingesetzt, d. h. auf ein Mol des Sulfenylchlorids der Formel VI setzt man vorzugsweise etwa 2 Mol des Carbamoyloxims der Formel I' ein.

Die Isolierung der so erhaltenen Verbindung der Formel I erfolgt in üblicher Weise.

Sofern keine gezielte Synthese zur Isolierung reiner Isomerer durchgeführt wird, fällt normalerweise ein Produkt als Gemisch zweier oder mehrerer Isomerer an. Die Isomeren können nach an sich bekannten Methoden aufgetrennt werden.

Die als Ausgangsmaterialien in den Verfahrensvarianten a), b) und c) verwendbaren Oxime der Formel II sind bislang noch nicht bekannt ; sie können aber auf einfache Weise hergestellt werden, indem man ein Thiazolin der Formel

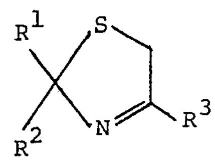

(VII)

worin $R^1$, $R^2$ und $R^3$ die oben angegebenen Bedeutungen besitzen, zweckmässigerweise in Gegenwart eines Verdünnungsmittels nitrosiert.

Nitrosiert wird zweckmässigerweise nach an sich bekannten Methoden, also mittels :

Nitrit, z. B. Alkalimetallnitrit, wie Natriumnitrit, und Säure, z. B. Essigsäure, Propionsäure, ein Gemisch von Essigsäure und Essigsäureanhydrid oder eine wässrige Mineralsäure, wie Salzsäure ;

Stickstofftetroxid ; Nitrosylchlorid ; Alkylnitrit, z. B. Methyl-, Aethyl- oder Isopentylnitrit ; diese letzteren Nitrosoverbindungen können sowohl in saurem als auch in alkalischem Medium umgesetzt werden.

Als Verdünnungsmittel eignen sich insbesondere Wasser, Alkohole, Acetonitril, aromatische Kohlenwasserstoffe, chlorierte Kohlenwasserstoffe sowie Aether.

Die Reaktionstemperaturen liegen zweckmässigerweise zwischen – 20 °C und 100 °C, vorzugsweise zwischen 0 °C und 60 °C.

Zur Isolierung der zo erhaltenen Verbindung der Formel II kann das Lösungsmittel entfernt werden, z. B. durch Abfiltrieren oder Abdestillieren, und der Rückstand entweder gewaschen, z. B. mit Wasser, getrocknet und kristallisiert bzw. chromatographisch gereinigt werden, oder in ein geeignetes Lösungsmittel aufgenommen, mit Wasser gewaschen, die Lösung über einem geeigneten Trocknungsmittel, z. B. wasserfreiem Natriumsulfat, getrocknet und nach Abdestillieren des Lösungsmittels das Produkt umkristallisiert oder chromatographisch gereinigt werden.

Die als Ausgangsmaterialien verwendbaren Thiazoline der Formel VII sind entweder bekannt oder können nach an sich bekannten Methoden, z. B. gemäss F. Asinger et al., Ann. 610, 17-24 und 33-49 (1957), Angew. Chemie 79, 953 (1967) oder DOS 2 645 731, hergestellt werden.

Die als Ausgangsmaterialien in den Verfahrensvarianten a), b) und c) verwendbaren Oxime der Formel II können auch aus Glyoxylhydroxamsäurechlorid oder dessen Alkyl- oder Cycloalkylderivat und dem entsprechenden Mercaptoamin der Formel

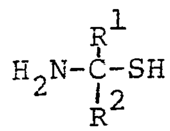

hergestellt werden, und zwar nach dem folgenden Verfahrensschema :

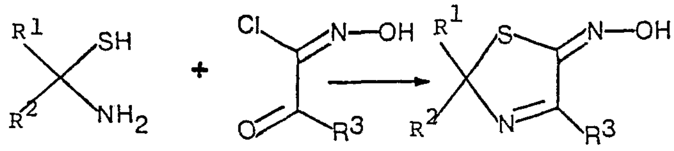

(VIII) (IX) (II)

Die Umsetzung wird zweckmässigerweise in einem inerten Verdünnungsmittel, in Gegenwart eines säurebindenden Mittels und in einem Temperaturbereich zwischen − 30 °C und der Raumtemperatur durchgeführt. Als inerte Verdünnungsmittel eignen sich insbesondere polare Lösungsmittel, wie beipielsweise Aether und ätherartige Verbindungen, z. B. Diäthyläther und Tetrahydrofuran ; Pyridin ; Acetonitril ; Alkohole ; und Wasser. Als säurebindende Mittel kommen insbesondere Alkalicarbonate, wie beispielsweise Natriumcarbonat, Kaliumcarbonat und Natriumhydrogencarbonat ; niedere tertiäre Alkylamine, wie beispielsweise Triäthylamin ; und Pyridin in Frage.

Die Isocyanate der Formel III sind entweder bekannt oder können nach an sich bekannten Methoden, z. B. durch Umsetzung des diesbezüglichen Amins $R^5NH_2$ mit Phosgen und anschliessendes Erhitzen, hergestellt werden. Beispiele solcher bekannter Isocyanate sind Methylisocyanat, Aethylisocyanat, Isopropylisocyanat, tert.-Butylisocyanat, Allylisocyanat, Cyclopropylisocyanat und Cyclohexylisocyanat.

Die Carbamoylhalogenide der Formel IV sind ebenfalls entweder bekannt oder können nach an sich bekannten Methoden, z. B. durch Umsetzung des diesbezüglichen Amins $R^4R^5NH$ mit Phosgen, hergestellt werden. Beispiele solcher bekannter Carbamoylhalogenide sind Dimethylcarbamoylchlorid, Methyläthylcarbamoylchlorid, N-Methyl-N-trichlormethylsulfenyl-carbamoylfluorid, N-Methyl-N-fluordichlormethylsulfenyl-carbamoylfluorid, N-Methyl-N-chlordifluormethylsulfenyl-carbamoylfluorid, N-Methyl-N-(3-trifluormethylphenylsulfenyl)-carbamoylfluorid, N-Acetyl-N-methylcarbamoylchlorid, N-Methyl-N-(4-chlorphenylsulfenyl)-carbamoylfluorid, N-Methyl-N-(morpholin-1-ylsulfenyl)-carbamoylfluorid und N-Methyl-N-(tert.-butylthio)sulfenylcarbamoylfluorid.

Die Amine der Formel V sind ebenfalls entweder bekannt oder können nach an sich bekannten Methoden hergestellt werden. Beispiele solcher bekannter Amine sind Methylamin, Aethylamin, Dimethylamin, Methyläthylamin und Allylmethylamin.

Die Sulfenylchloride der Formel VI sind ebenfalls entweder bekannt oder können nach an sich bekannten Methoden hergestellt werden. Beispiele solcher bekannter Sulfenylchloride sind Methylsulfenylchlorid, Trichlormethylsulfenylchlorid, Dichlorfluormethylsulfenylchlorid, Chlordifluormethylsulfenylchlorid, Trifluormethylsulfenylchlorid, Phenylsulfenylchlorid, 2,4-Dichlorphenylsulfenylchlorid, 3-Trifluormethylphenylsulfenylchlorid, 3-Methylphenylsulfenylchlorid, 4-Chlor-3-trifluormethylphenylsulfenylchlorid, Schwefeldichlorid und Schwefelmonochlorid.

Die Verbindungen der Formel I sind ganz allgemein als Pestizide von Wert. Sie zeigen sich besonders wertvoll zur Bekämpfung von Insekten, Milben, Nematoden und Mollusken, insbesondere gegen

— Coleoptera wie z. B. Epilachna spp., Leptinotarsa decemlineata, Anthonomus spp., Conotrachelus nenuphar, Lema spp., Lissorhoptrus oryzaephilus, Phyllotreta spp., Psylliodes chrysocephala, Meligethes aenus, Ceutorrhynchus assimilis, Agriotes spp., Otiorhynchus sulcatus, Melolontha melolontha und Diabrotica spp.

— Lepidoptera wie z. B. Laspeyresia spp., Adoxophyes orana, Tortrix viridana, Cheimatobia brumata, Lyonetia clerkella, Operophtera brumata, Lithocolletis blancardella, Porthetria dispar, Mamestra brassicae, Agrotis segetum, Plutella spp., Pieris brassicae, Choristoneura fumiferana, Heliothis spp., Spodoptera spp., Pectinophora gossipiella, Chilo spp., Ostrinia nubilalis, Clysia ambiguella, Lobesia botrana.

— Diptera wie z. B. Drosophila melanogaster, Ceratitis spp., Oscinella frit, Dacus spp. und Rhagoletis spp. Leatherjacket spp., Sciara spp., Phorbia spp. und Megasetia agarici.

— Homoptera, d. h. Blattläuse wie z. B. Aphis fabae, Myzus persicae und weitere Arten dieser Gattungen, Rhophalosiphon spp., Schizaphis spp., Dysaphis spp., Eriosoma spp., Macrosiphum spp., Adelges spp., Sitobion avenae, Metopolophium spp. sowie Schild- und Schmierläuse wie z. B. Aspidiotus spp., Saissetia spp., Quadraspidiotus perniciosus, Aonidiella aurantii, Coccus spp., Lepidosaphes spp., Planococcus spp., Pseudococcus spp., Ceroplastes spp., Icerya purchasi, Chrysomphalus spp., Parlatoria spp., Rhizoecus spp. sowie Zikaden wie z. B. Nephotettix spp., Laodelphax spp., Nilaparvata spp., Sogatella spp. und Erythroneura spp., Aleurodidae wie z. B. Trialeurodes vaporariorum, Dialeurodes spp., Aleurothrixus spp., Bemisia spp., Aleyrodes spp., ausserdem Trips-Arten und Wanzen.

— Acarina wie z. B. Tetranychus urticae, Panonychus ulmi und andere Tetranychiden, Tarsonemiden wie Steneotarsonemus spp., Tenuipalpiden wie Brevipalpus, Eriophyiden wie Phyllocoptruta oleivora, Aceria sheldoni, Eriophyes spp., Aceria spp. und ferner Zecken.

— Nematoda wie z. B. freilebende Nematoden (u. a. Pratylenchus spp. wie P. penetrans), blattparasitische Nematoden (u. a. Aphelenchoides) und wurzelparasitische Nematoden (u. a. Meloidogynae spp. wie M. incognita, Globodera spp. wie G. rostochiensis).

— Mollusca wie z. B. Deroceras spp.

Die erfindungsgemässen Verbindungen wirken als Kontakt- und Frassgifte und sind systemisch wirksam.

Das erfindungsgemässe Schädlingsbekämpfungsmittel ist dadurch gekennzeichnet, dass es eine wirksame Menge von mindestens einer Verbindung der Formel I, wie oben definiert, sowie inertes Trägermaterial enthält.

Die erfindungsgemässen Wirkstoffe der Formel I können in die üblichen Formulierungen übergeführt werden, wie Lösungen, Emulsionen, Suspensionen, Pulver, Pasten und Granulate. Diese werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln, Netzmitteln und/oder Dispergiermitteln. Im Falle der Benutzung von Wasser als Streckmittel können z. B. auch organische Lösungsmittel als Hilfslösungsmittel verwendet werden.

Als flüssige Lösungsmittel kommen im wesentlichen in Frage : Aromaten wie Toluol, Xylole und Alkylnaphthaline ; chlorierte Aromaten und chlorierte aliphatische Kohlenwasserstoffe wie Chlorbenzole, Chloräthylene und Methylenchlorid ; aliphatische Kohlenwasserstoffe wie Cyclohexan und Paraffine, z. B. Erdölfraktionen ; Alkohole, wie Butanol und Glykol, sowie deren Aether und Ester ; Ketone wie Aceton, Methyläthylketon, Methylisobutylketon und Cyclohexanon ; und stark polare Lösungsmittel wie Dimethylformamid, N-Methylpyrrolidon und Dimethylsulfoxid sowie Wasser. Unter verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase wie Halogenkohlenwasserstoffe, z. B. Dichlordifluormethan.

Als feste Trägerstoffe kommen im wesentlichen in Frage : natürliche Mineralstoffe wie Kaoline, Tonerden, Talkum, Kreide, Quarz, Attapulgit, Montmorillonit und Diatomeenerde ; und synthetische Mineralstoffe wie hochdisperse Kieselsäure, Aluminiumoxyd und Silikate.

Als oberflächenaktive Mittel, insbesondere Emulgiermittel und Netzmittel, kommen in Betracht : nichtionogene und anionische Emulgatoren wie Polyoxyäthylen-Fettsäure-Ester, Polyoxyäthylen-Fettalkohol-Aether, Alkylarylpolyglykoläther, Alkylsulfonate, Alkylsulfate und Arylsulfonate ; und als Dispergiermittel : z. B. Lignin, Sulfitablaugen und Methylcellulose.

Die erfindungsgemässen Schädlingsbekämpfungsmittel enthalten im allgemeinen zwischen 0,000 5 und 95 Gewichtsprozent der Verbindung bzw. Verbindungen der Formel I als Wirkstoff, vorzugsweise zwischen 1 und 75 Gewichtsprozent. Sie können in einer Form vorliegen, die sich für die Lagerung und den Transport eignet. In solchen Formen ist die Wirkstoffkonzentration normalerweise im höheren Bereich der obigen Konzentrationsreihe. Diese Formen können dann mit gleichen oder verschiedenen Trägermaterialien bis zu Wirkstoffkonzentrationen verdünnt werden, die sich für den praktischen Gebrauch eignen, und solche Konzentrationen liegen normalerweise im niedrigeren Bereich der obigen Konzentrationsreihe. Als Anwendungsformen kommen u. a. gebrauchsfertige Lösungen, Emulsionen, Suspensionen, Schäume, Pulver, Pasten, Stäubemittel und Granulate in Frage, deren Anwendung in üblicher Weise geschieht, z. B. durch Verspritzen, Versprühen, Vernebeln, Verstäuben, Verstreuen, Eindrillen, Verräuchern, Giessen, Beizen oder Inkrustieren.

Die Wirkstoffkonzentrationen in den anwendungsfertigen Zubereitungen können in grossen Bereichen variiert werden. In Spritzbrühen können z. B. Konzentrationen zwischen 0,000 5 und 20 Gewichtsprozent vorliegen. Im Ultra-Low-Volume-Verfahren können Spritzbrühen formuliert werden, in denen die Wirkstoffkonzentration vorzugsweise von 10 bis 20 Gewichtsprozent beträgt, während die im Low-Volume-Verfahren und im High-Volume-Verfahren formulierten Spritzbrühen vorzugsweise eine Wirkstoffkonzentration von 0,5 bis 1,0 bzw. 0,05 bis 0,1 Gewichtsprozent aufweisen. Granulate enthalten vorzugsweise von 2 bis 10 Gewichtsprozent der Verbindung bzw. Verbindungen der Formel I als Wirkstoff.

Die erfindungsgemässen Schädlingsbekämpfungsmittel können neben den Wirkstoffen der Formel I auch andere Wirkstoffe enthalten, z. B. anderweitige Schädlingsbekämpfungsmittel. Solche Kombinationsmittel eignen sich zur Verstärkung der Aktivität bzw. zur Verbreiterung des Wirkungsspektrums.

Zur Herstellung von den erfindungsgemässen Schädlingsbekämpfungsmitteln wird der Wirkstoff der Formel I mit inertem Trägermaterial vermischt. Im Falle von pulverförmigen Mitteln kann der Wirkstoff mit den Trägerstoffen vermischt werden, z. B. durch Zusammenmahlen ; oder man kann das inerte Trägermaterial mit einer Lösung oder Suspension des Wirkstoffs imprägnieren und dann die Lösungs- oder Suspensionsmittel durch Abdunsten, Erhitzen oder durch Absaugen unter vermindertem Druck entfernen. Durch Zusatz von Netz- und/oder Dispergiermitteln kann man solche pulverförmige Mittel mit Wasser leicht benetzbar machen, so dass sie in wässrige Suspensionen, die sich z. B. als Spritzmittel eignen, übergeführt werden können. Zur Herstellung emulgierbarer Konzentrate, die sich insbesondere für die Lagerung und den Transport eignen, kann der Wirkstoff beispielsweise mit einem Emulgiermittel gemischt oder auch in einem inerten Lösungsmittel gelöst und danach mit einem Emulgator gemischt werden. Durch Verdünnen solcher Konzentrate mit Wasser erhält man gebrauchsfertige Emulsionen.

Die erfindungsgemässen Schädlingsbekämpfungsmittel werden dadurch verwendet, dass man die zu schützenden Gegenstände oder die Schädlinge selbst mit dem erfindungsgemässen Schädlingsbekämpfungsmittel behandelt. Dieses Anwendungsverfahren wird vorzugsweise durch Boden- oder Blattapplikation, je nach Art der zu bekämpfenden Schädlinge, durchgeführt.

Die nachfolgenden Beispiele dienen zur näheren Erläuterung der Erfindung.

I. Herstellung der Wirkstoffe :

## Beispiel 1

20,0 g (0,126 Mol) 5-Oxo-2,2,4-trimethyl-3-thiazolinoxim werden in 100 ml Chloroform gelöst. Zu der Lösung gibt man 15 ml (0,252 Mol) frisch destilliertes Methylisocyanat sowie 3 Tropfen Triäthylamin hinzu. Das Gemisch, das sich nach kurzer Zeit leicht erwärmt, wird 16 Stunden bei Raumtemperatur stehen gelassen. Man filtriert anschliessend die schwach trübe Lösung und destilliert das Lösungsmittel und überschüssiges Methylisocyanat im Vakuum ab. der ölige Rückstand wird an 250 g Kieselgel mit Aethylacetat/n-Hexan (1 : 1) als Laufmittel filtriert, das Lösungsmittel durch Eindampfen entfernt und der entstehende Rückstand aus Methylenchlorid/n-Hexan/Diäthyläther umkristallisiert. Man erhält als weisse Kristalle, Fp. 90-92 °C, 5-Oxo-2,2,4-trimethyl-3-thiazolin-O-(methylcarbamoyl)oxim.

## Beispiel 2

3,0 g (0,019 Mol) 5-Oxo-2,2,4-trimethyl-3-thiazolinoxim werden portionenweise zu einer Suspension von 0,9 g Natriumhydrid in 20 ml Tetrahydrofuran gegeben. (Das Natriumhydrid selbst wurde kurz vor seiner Verwendung aus dessen 55 %iger Suspension in Oel durch zweimaliges Extrahieren mit wenig absolutem Pentan freigesetzt.) Nach beendeter Wasserstoffentwicklung wird 1 Stunde bei Raumtemperatur nachgerührt. Man tropft 2,05 g (0,019 Mol) Dimethylcarbamoylchlorid zu und lässt das Reaktionsgemisch 16 Stunden bei Raumtemperatur nachrühren. Der Rückstand wird dann in Diäthyläther aufgenommen und die organische Phase mit gesättigter Natriumchloridlösung gewaschen und über wasserfreiem Natriumsulfat getrocknet. Anschliessend destilliert man das Lösungsmittel ab und lässt den Rückstand aus Diäthyläther/n-Hexan umkristallisieren. Man erhält 5-Oxo-2,2,4-trimethyl-3-thiazolin-O-(dimethylcarbamoyl) oxim, Fp. 92-95 °C.

## Beispiel 3-22

Analog dem in Beispiel 1 beschriebenen Verfahren werden die entsprechenden Ausgangsmaterialien der Formeln II und III umgesetzt, um die in der nachstehenden Tabelle A aufgeführten Verbindungen der Formel I herzustellen.

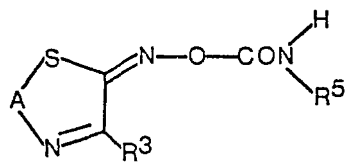

Tabelle A

| Beispiel | A | $R^3$ | $R^5$ | Fp. |
|---|---|---|---|---|
| 3 | $(CH_3)(C_2H_5)C<$ | $CH_3$ | $CH_3$ | -(Oel) |
| 4 | $(C_2H_5)(n-C_4H_9)C<$ | $CH_3$ | $CH_3$ | -(Oel) |
| 5 | $(CH_2)_4C<$ | $CH_3$ | $CH_3$ | 117-119°C |
| 6 | $CH_3CH<$ | $CH_3$ | $CH_3$ | 112-114°C |
| 7 | $C_2H_5CH<$ | $CH_3$ | $CH_3$ | 109-110°C |
| 8 | $(CH_3)_2CHCH<$ | $CH_3$ | $CH_3$ | 120-123°C |
| 9 | $(CH_3)(C_2H_5)C<$ | H | $CH_3$ | 95- 96°C |
| 10 | $(CH_3)(CH_3)C<$ | $C_2H_5$ | $CH_3$ | 102-105°C |
| 11 | $(CH_3)(CH_3)C<$ | $(CH_3)_2CH$ | $CH_3$ | 94- 96°C |
| 12 | $(CH_3)(CH_3)C<$ | $CH_3$ | $C_2H_5$ | 95- 97°C |
| 13 | $(CH_3)(C_2H_5)C<$ | $CH_3$ | $(CH_3)_2CH$ | 80- 81°C |
| 14 | $(CH_3)(CH_3)C<$ | 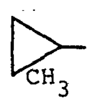 | $CH_3$ | 161-163°C |
| 15 | ( 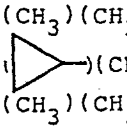 )$(CH_3)C<$ | $CH_3$ | $CH_3$ | 77-79°C |
| 16 | $(CH_3)(CH_3)C<$ | $CH_3$ | $CH_2CH=CH_2$ | 46-48°C |
| 17 | $(CH_3)(CH_3)C<$ | $CH_3$ | 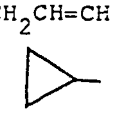 | 77-78°C |
| 18 | $(C_2H_5)(C_2H_5)C<$ | $CH_3$ | $CH_3$ | 48-50°C |

Beispiel 19

3,2 g (0,015 Mol) 5-Oxo-2,2,4-trimethyl-3-thiazolin-O-(methylcarbamoyl)oxim werden in 15 ml absolutem Methylenchlorid gelöst und bei 0 °C unter gutem Rühren tropfenweise mit 1,2 ml Pyridin und 0,55 ml (0,086 Mol) frisch destilliertem Schwefeldichlorid versetzt. Man lässt das Gemisch langsam Raumtemperatur erreichen und rührt 16 Stunden nach. Das Gemisch wird anschliessend mit 100 ml Methylenchlorid verdünnt, einmal mit verdünnter Salzsäure und zweimal mit halbgesättigter Natriumchloridlösung gewaschen und über wasserfreiem Natriumsulfat getrocknet. Danach destilliert man das Lösungsmittel ab und reinigt den öligen Rückstand mittels zweimaliger Chromatographie an Silicagel (Laufmittel 7 % Aceton in Chloroform ; 40 % Toluol in Aethylacetat) und Umkristallisierens aus Aceton/n-Hexan. Man erhält N,N'-Bis-[2,2,4-trimethyl-3-thiazolin-5-O-(methylcarbamoyl)oximino] sulfid, Fp. 152-154 °C.

Beispiel 20

3,0 g (0,019 Mol) 5-Oxo-2,2,4-trimethyl-3-thiazolinoxim werden in 10 ml absolutem Acetonitril suspendiert. Zu der Suspension bei 0 °C werden nacheinander 5,5 g wasserfreies Kaliumcarbonat und, tropfenweise, eine Lösung von 4,52 g N-(Di-n-butylaminosulfenyl)-N-methylcarbamoylfluorid in 10 ml absolutem Acetonitril gegeben. Man lässt das Reaktionsgemisch 20 Stunden bei Raumtemperatur gut rühren. Anschliessend filtriert man die ausgefallenen Kaliumsalze ab und dampft das Filtrat ein. Chromatographie des so erhaltenen öligen Rohproduktes an Silicagel mit Aethylacetat/Toluol (1 : 3) als Laufmittel liefert 5-Oxo-2,2,4-trimethyl-3-thiazolin-O-[N-(di-n-butylaminosulfenyl)-N-methylcarbamoyl] oxim, $n_D^{20}$ 1,520 0.

Beispiele 21-25

Analog dem in Beispiel 20 beschriebenen Verfahren werden 5-Oxo-2,2,4-trimethyl-3-thiazolin-oxim und das entsprechende Ausgangsmaterial der Formel IV umgesetzt, um die in der nachstehenden Tabelle B aufgeführten Verbindungen der Formel I herzustellen.

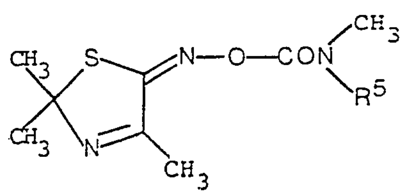

Tabelle B

| Beispiel | $R^5$ | Fp. | $n_D^{20}$ |
|---|---|---|---|
| 21 | 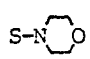 | 103-104°C | - |
| 22 | $S-S-CH(CH_3)_2$ | - | 1,5590 |
| 23 | $S-S-C(CH_3)_3$ | 74-76°C | - |
| 24 | $S-CCl_3$ | 90-93°C | - |
| 25 | 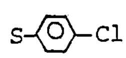 | 144-146°C | - |

II. Herstellung der Ausgangsmaterialien

Beispiel 26

Das als Ausgangsmaterial in den Beispielen 1, 2, 12, 16, 17 und 20-25 benötigte 5-Oxo-2,2,4-trimethyl-3-thiazolin-oxim kann wie folgt hergestellt werden :

30,0 g (0,232 Mol) 2,2,4-Trimethyl-3-thiazolin werden in 200 ml Acetonitril gelöst. In der Lösung suspendiert man 32,0 g (0,46 Mol) Natriumnitrit. Dann tropft man zu der auf − 10 °C gekühlten Suspension unter gutem Rühren 26,6 ml (0,464 Mol) Essigsäure und anschliessend 21,9 ml (0,232 Mol)

Essigsäureanhydrid zu. Nach beendeter Zugabe lässt man das auf 0 °C erwärmte Reaktionsgemisch noch 3 Stunden bei Raumtemperatur nachrühren.

Man engt dann das Reaktionsgemisch im Vakuum etwas ein, versetzt den Rückstand mit ca. 500 ml Wasser und gibt zum Gemisch genug festes Natriumhydrogencarbonat zu, bis pH 6 erreicht wird. Nach dreimaligem Extrahieren mit je 200 ml Chloroform wäscht man die Extrakte mit Wasser, trocknet die organische Phase über wasserfreiem Natriumsulfat und engt die Phase im Vakuum ein, bis sie trüb wird. Man filtriert die organische Phase und versetzt das Filtrat mit n-Hexan. Das 5-Oxo-2,2,4-trimethyl-3-thiazolin-oxim fällt als hellbraunes Kristallisat aus und wird abfiltriert und zweimal aus Chloroform/n-Hexan umkristallisiert. Man erhält ein Produkt, das bei 161-163 °C schmilzt.

## Beispiel 27

Das 5-Oxo-2,2,4-trimethyl-3-thiazolin-oxim kann auch wie folgt hergestellt werden :

30,0 g (0,232 Mol) 2,2,4-Trimethyl-3-thiazolin und 17,6 g (0,255 Mol) Natriumnitrit werden in 75 ml Wasser gelöst und unter gutem Rühren bei 45 °C tropfenweise mit 26,6 ml (0,464 Mol) Essigsäure versetzt. Man lässt die Innentemperatur des Reaktionsgemisches langsam auf 10-15 °C steigen und rührt dann bei dieser Temperatur noch 2 Stunden. Das Produkt fällt als ockerbraunes Kristallisat an und wird filtriert, mit Wasser gewaschen und im Vakuum getrocknet. Nach einmaligem Umkristallisieren aus Chloroform/n-Hexan erhält man 5-Oxo-2,2,4-trimethyl-3-thiazolinoxim, Fp. 159-161 °C.

## Beispiele 28-39

Analog dem in Beispiel 26 oder 27 beschriebenen Verfahren werden die entsprechenden substituierten 3-Thiazoline mit Natriumnitrit umgesetzt, um die der nachstehenden Tabelle C aufgeführten Ausgangsmaterialien der Formel II herzustellen. Die entsprechenden Endprodukte der Formel I sind auch in dieser Tabelle angegeben.

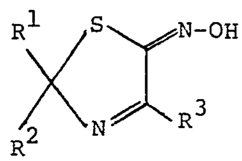

Tabelle C

| Beispiel | $R^1$ | $R^2$ | $R^3$ | Fp. | Beispiel-Nr. des Endproduktes der Formel I |
|---|---|---|---|---|---|
| 28 | $CH_3$ | $C_2H_5$ | $CH_3$ | - (nicht gereinigt) | 3, 13 |
| 29 | $C_2H_5$ | $n-C_4H_9$ | $CH_3$ | -(Oel) | 4 |
| 30 | $-(CH_2)_4-$ | | $CH_3$ | 157-159°C | 5 |
| 31 | H | $CH_3$ | $CH_3$ | 145-148°C | 6 |
| 32 | H | $C_2H_5$ | $CH_3$ | 115-118°C | 7 |
| 33 | H | $(CH_3)_2CH$ | $CH_3$ | - (nicht gereinigt) | 8 |
| 34 | $CH_3$ | $C_2H_5$ | H | - (nicht gereinigt) | 9 |
| 35 | $CH_3$ | $CH_3$ | $C_2H_5$ | - | 10 |

Tabelle C (Fortsetzung)

| Beispiel | $R^1$ | $R^2$ | $R^3$ | Fp. | Beispiel-Nr. des Endproduktes der Formel I |
|---|---|---|---|---|---|
| | | | | (nicht gereinigt) | |
| 36 | $CH_3$ | $CH_3$ | $(CH_3)_2CH$ | 187-192°C | 11 |
| 37 | $CH_3$ | $CH_3$ | 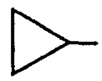 | 180-181,5°C | 14 |
| 38 | $CH_3$ | 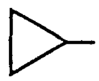 | $CH_3$ | - (nicht gereinigt) | 15 |
| 39 | $C_2H_5$ | $C_2H_5$ | $CH_3$ | - (nicht gereinigt) | 18 |

III. Formulierungsbeispiele

Beispiel 40

Die Verbindungen der Formel I können beispielsweise wie folgt formuliert werden :

| | g/Liter |
|---|---|
| a) Wirkstoff der Formel I | 250 |
| N-Methyl-2-pyrrolidon | 500 |
| Emulgator-Mischung bestehend aus : | |
| Calcium-Alkylarylsulfonat, Alkylphenoläthoxylat, Blockpolymerisat aus Propylenoxid und Aethylenoxid | 50 |
| Calcium-Dodecylbenzolsulfonat | 25 |
| Lösungsmittel (Gemisch aus Mono-, Di- und Tri- nieder Alkylbenzolen) ad | 1 000 ml |

| | g/Liter |
|---|---|
| b) Wirkstoff der Formel I | 500 |
| N-Methyl-2-pyrrolidon ad | 1 000 ml |

| | |
|---|---|
| c) Wirkstoff der Formel I | 50 g |
| Hochdisperse Kieselsäure | 5 g |
| Natrium-Laurylsulfat | 1 g |
| Natrium-Lignosulfonat | 2 g |
| Kaolin | 42 g |
| | 100 g |

**Ansprüche** (für die Vertragsstaaten : BE, CH, DE, FR, GB, IT, LI, NL)

1. Verbindungen der allgemeinen Formel

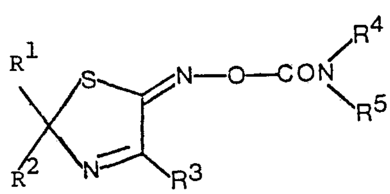

(I)

$R^1$ und $R^2$ Wasserstoff, $C_{1-4}$-Alkyl oder $C_{3-6}$-Cycloalkyl, oder zusammen mit dem Kohlenstoffatom, an das sie geknüpft sind, einen 4- bis 6-gliedrigen, gesättigten Kohlenwasserstoffring,
$R^3$ Wasserstoff, $C_{1-4}$-Alkyl oder $C_{3-6}$-Cycloalkyl,
$R^4$ Wasserstoff und
$R^5$ $C_{1-4}$-Alkyl, $C_{2-5}$-Alkenyl oder $C_{3-6}$-Cycloalkyl, oder
$R^4$ $C_{1-4}$-Alkyl und
$R^5$ $C_{1-4}$-Alkyl, ($C_{1-4}$-Alkyl)carbonyl, Tri-($C_{1-4}$-Alkyl)silyl oder eine Gruppe (a)

$$—S_m—R^6 \qquad (a)$$

$R^6$ $C_{1-8}$-Alkyl ; $C_{1-4}$-Halogenalkyl ; Phenyl, gegebenenfalls mit Halogen, $C_{1-4}$-Alkyl und/oder Trifluormethyl substituiert ; oder eine Gruppe (b) oder (c)

$$—NR^7R^8$$

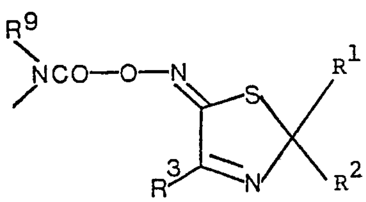

(c)

$R^7$ und $R^8$ $C_{1-4}$-Alkyl oder $C_{1-4}$-Halogenalkyl, oder zusammen mit dem Stickstoffatom, an das sie geknüpft sind, eine Piperidino- oder Morpholinogruppe,
$R^9$ $C_{1-4}$-Alkyl und m 1 oder 2 bedeuten.

2. Verbindungen nach Anspruch 1, worin $R^1$ Wasserstoff oder $C_{1-4}$-Alkyl bedeutet.

3. Verbindungen nach Anspruch 1 oder 2, worin $R^2$ Wasserstoff oder $C_{1-4}$-Alkyl bedeutet.

4. Verbindungen nach einem der Ansprüche 1 bis 3, worin $R^3$ Methyl bedeutet.

5. Verbindungen nach einem der Ansprüche 1 bis 4, worin $R^4$ Wasserstoff und $R^5$ $C_{1-4}$-Alkyl bedeuten.

6. 5-Oxo-2,2,4-trimethyl-3-thiazolin-O-(methylcarbamoyl)oxim.

7. Verbindungen nach Anspruch 1 als Schädlingsbekämpfungsmittel.

8 Schädlingsbekämpfungsmittel, dadurch gekennzeichnet, dass es eine wirksame Menge mindestens einer Verbindung der allgemeinen Formel I in Anspruch 1 sowie inertes Trägermaterial enthält.

9. Schädlingsbekämpfungsmittel nach Anspruch 8, dadurch gekennzeichnet, dass es eine wirksame Menge von 5-Oxo-2,2,4-trimethyl-3-thiazolin-O-(methylcarbamoyl)oxim enthält.

10. Verfahren zur Herstellung von denjenigen Verbindungen der allgemeinen Formel I in Anspruch 1, worin $R^4$ Wasserstoff und $R^5$ $C_{1-4}$-Alkyl, $C_{2-5}$-Alkenyl oder $C_{3-6}$-Cycloalkyl bedeuten, dadurch gekennzeichnet, dass man ein Oxim der Formel

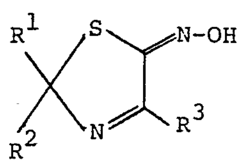

(II)

worin $R^1$, $R^2$ und $R^3$ die in Anspruch 1 angegebenen Bedeutungen besitzen, mit einem Isocyanat der Formel

$$R^5—N = C = O \qquad (III)$$

worin $R^5$ die oben angegebene Bedeutung besitzt, umsetzt.

11. Verfahren zur Herstellung von Verbindungen der allgemeinen Formel I in Anspruch 1, dadurch gekennzeichnet, dass man ein Oxim der Formel II in Anspruch 10 mit einem Carbamoylhalogenid der Formel

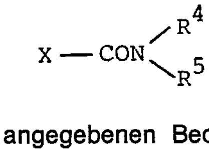

(IV)

worin $R^4$ und $R^5$ die in Anspruch 1 angegebenen Bedeutungen besitzen und X ein Halogenatom bedeutet, umsetzt.

12. Verfahren zur Herstellung von Verbindungen der allgemeinen Formel I in Anspruch 1, dadurch gekennzeichnet, dass man ein Oxim der Formel II in Anspruch 10 mit Phosgen und anschliessend einem Amin der Formel

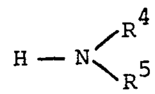

(V)

worin $R^4$ und $R^5$ die in Anspruch 1 angegebenen Bedeutungen besitzen, umsetzt.

13. Verfahren zur Herstellung von denjenigen Verbindungen der allgemeinen Formel I in Anspruch 1, worin $R^4$ $C_{1-4}$-Alkyl und $R^5$ eine Gruppe (a) bedeuten, dadurch gekennzeichnet, dass man ein gegebenenfalls nach einem der Ansprüche 10 bis 12 erhaltenes Carbamoyloxim der Formel

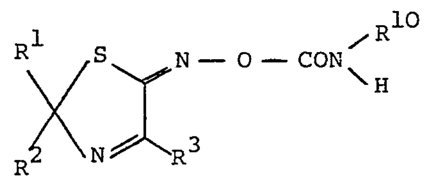

(I')

worin $R^1$, $R^2$ und $R^3$ die in Anspruch 1 angegebenen Bedeutungen besitzen und $R^{10}$ $C_{1-4}$-Alkyl bedeutet, mit einem Sulfenylchlorid der Formel

$$Cl—S_m—R^{11} \qquad (VI)$$

worin m 1 oder 2 und $R^{11}$ eine der in Anspruch 1 angegebenen Bedeutungen von $R^6$ oder Chlor bedeuten, umsetzt.

14. Verwendung einer der in den Ansprüchen 1 bis 7 genannten Verbindungen bzw. eines in Anspruch 8 genannten Mittels zur Bekämpfung von Schädlingen.

15. Verbindungen der allgemeinen Formel

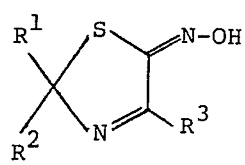

(II)

worin

$R^1$ und $R^2$ Wasserstoff, $C_{1-4}$-Alkyl oder $C_{3-6}$-Cycloalkyl, oder zusammen mit dem Kohlenstoffatom, an das sie geknüpft sind, einen 4- bis 6-gliedrigen, gesättigten Kohlenwasserstoffring und

$R^3$ Wasserstoff, $C_{1-4}$-Alkyl oder $C_{3-6}$-Cycloalkyl bedeuten.

16. Verbindungen nach Anspruch 15, worin $R^1$ Wasserstoff oder $C_{1-4}$-Alkyl bedeutet.

17. Verbindungen nach Anspruch 15 oder 16, worin $R^2$ Wasserstoff oder $C_{1-4}$-Alkyl bedeutet.

18. Verbindungen nach einem der Ansprüche 15 bis 17, worin $R^3$ Methyl bedeutet.

19. 5-Oxo-2,2,4-trimethyl-3-thiazolin-oxim.

20. Verfahren zur Herstellung von Verbindungen der Formel II in Anspruch 15, dadurch gekennzeichnet, dass man eine Verbindung der Formel

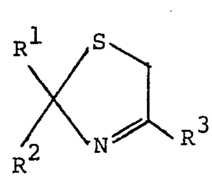

(VII)

worin $R^1$, $R^2$ und $R^3$ die in Anspruch 15 angegebenen Bedeutungen besitzen, nitrosiert.

21. Verfahren zur Herstellung von Verbindungen der Formel II in Anspruch 15, dadurch gekennzeichnet, dass man eine Verbindung der Formel

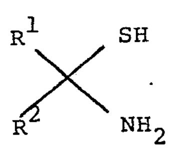

(VIII)

worin $R^1$ und $R^2$ die in Anspruch 15 angegebenen Bedeutungen besitzen, mit einer Verbindung der Formel

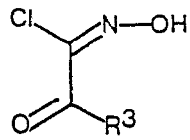

(IX)

worin $R^3$ die in Anspruch 15 angegebenen Bedeutungen besitzt, umsetzt.

**Ansprüche** (für den Vertragsstaat AT)

1. Schädlingsbekämpfungsmittel, dadurch gekennzeichnet, dass es eine wirksame Menge von mindestens einer Verbindung der allgemeinen Formel

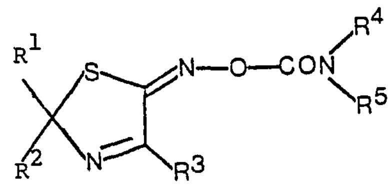

(I)

worin

$R^1$ und $R^2$ Wasserstoff $C_{1\text{-}4}$-Alkyl oder $C_{3\text{-}6}$-Cycloalkyl, oder zusammen mit den Kohlenstoffatom, an das sie geknüpft sind, einen 4- bis 6-gliedrigen, gesättigten Kohlenwasserstoffring,

$R^3$ Wasserstoff, $C_{1\text{-}4}$-Alkyl oder $C_{3\text{-}6}$-Cycloalkyl,

$R^4$ Wasserstoff, und

$R^5$ $C_{1\text{-}4}$-Alkyl, $C_{2\text{-}5}$-Alkenyl oder $C_{3\text{-}6}$-Cycloalkyl, oder

$R^4$ $C_{1\text{-}4}$-Alkyl, und

$R^5$ $C_{1\text{-}4}$-Alkyl, ($C_{1\text{-}4}$-Alkyl)carbonyl, Tri-($C_{1\text{-}4}$-Alkyl)silyl oder eine Gruppe (a)

$$—S_m—R^6 \qquad (a)$$

$R^6$ $C_{1\text{-}8}$-Alkyl ; $C_{1\text{-}4}$-Halogenalkyl ; Phenyl, gegebenenfalls mit Halogen, $C_{1\text{-}4}$-Alkyl und/oder Trifluormethyl substituiert ; oder eine Gruppe (b) oder (c)

$$—NR^7R^8 \qquad (b)$$

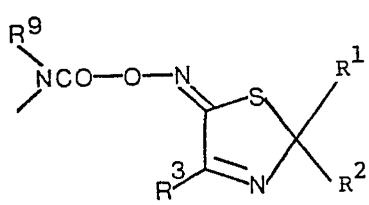

(c)

$R^7$ und $R^8$ $C_{1\text{-}4}$-Alkyl oder $C_{1\text{-}4}$-Halogenalkyl, oder zusammen mit dem Stickstoffatom, an das sie geknüpft sind, eine Piperidino- oder Morpholinogruppe,

$R^9$ $C_{1\text{-}4}$-Alkyl und

m 1 oder 2 bedeuten, sowie inertes Trägermaterial enthält.

2. Schädlingsbekämpfungsmittel nach Anspruch 1, worin $R^1$ Wasserstoff oder $C_{1\text{-}4}$-Alkyl bedeutet.

3. Schädlingsbekämpfungsmittel nach Anspruch 1 oder 2, worin $R^2$ Wasserstoff oder $C_{1\text{-}4}$-Alkyl bedeutet.

4. Schädlingsbekämpfungsmittel nach einem der Ansprüche 1 bis 3, worin $R^3$ Methyl bedeutet.

5. Schädlingsbekämpfungsmittel nach einem der Ansprüche 1 bis 4, worin $R^4$ Wasserstoff und $R^5$ $C_{1\text{-}4}$-Alkyl bedeuten.

6. Schädlingsbekämpfungsmittel nach Anspruch 1, dadurch gekennzeichnet, dass es eine wirksame Menge von 5-Oxo-2,2,4-trimethyl-3-thiazolin-O-(methylcarbamoyl)oxim sowie inertes Trägermaterial enthält.

7. Verfahren zur Herstellung von denjenigen Verbindungen der allgemeinen Formel I in Anspruch 1, worin $R^4$ Wasserstoff und $R^5$ $C_{1\text{-}4}$-Alkyl, $C_{2\text{-}5}$-Alkenyl oder $C_{3\text{-}6}$-Cycloalkyl bedeuten, dadurch gekennzeichnet, dass man ein Oxim der Formel

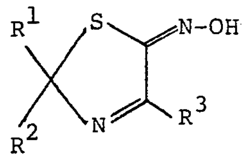

(II)

worin $R^1$, $R^2$ und $R^3$ die in Anspruch 1 angegebenen Bedeutungen besitzen, mit einem Isocyanat der Formel

$$R^5—N = C = O \qquad (III)$$

worin $R^5$ die oben angegebene Bedeutung besitzt, umsetzt.

8. Verfahren zur Herstellung von Verbindungen der allgemeinen Formel I in Anspruch 1, dadurch gekennzeichnet, dass man ein Oxim der Formel II in Anspruch 7 mit einem Carbamoylhalogenid der Formel

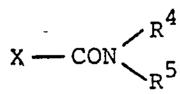

(IV)

worin $R^4$ und $R^5$ die in Anspruch 1 angegebenen Bedeutungen besitzen und X ein Halogenatom bedeutet, umsetzt.

9. Verfahren zur Herstellung von Verbindungen der allgemeinen Formel I in Anspruch 1, dadurch gekennzeichnet, dass man ein Oxim der Formel II in Anspruch 7 mit Phosgen und anschliessend einem Amin der Formel

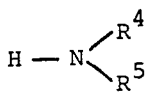

(V)

worin $R^4$ und $R^5$ die in Anspruch 1 angegebenen Bedeutungen besitzen, umsetzt.

10. Verfahren zur Herstellung von denjenigen Verbindungen der allgemeinen Formel I in Anspruch 1, worin $R^4$ $C_{1-4}$-Alkyl und $R^5$ eine Gruppe (a) bedeuten, dadurch gekennzeichnet, dass man ein gegebenenfalls nach einem der Ansprüche 7 bis 9 erhaltenes Carbamoyloxim der Formel

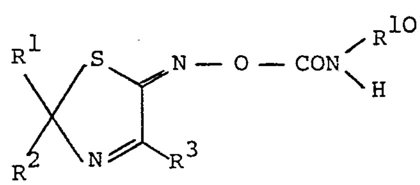

(I')

worin $R^1$, $R^2$ und $R^3$ die in Anspruch 1 angegebenen Bedeutungen besitzen und $R^{10}$ $C_{1-4}$-Alkyl bedeutet, mit einem Sulfenylchlorid der Formel

$$Cl—S_m—R^{11} \qquad (VI)$$

worin m 1 oder 2 und $R^{11}$ eine der in Anspruch 1 angegebenen Bedeutungen von $R^6$ oder Chlor bedeuten, umsetzt.

11. Verfahren zur Herstellung eines Schädlingsbekämpfungsmittels, dadurch gekennzeichnet, dass man mindestens eine der in den Ansprüchen 1 bis 6 genannten Verbindungen mit einem inerten Trägermaterial vermischt.

12. Verwendung einer bzw. eines der in den Ansprüchen 1 bis 6 genannten Verbindungen bzw. Mittel zur Bekämpfung von Schädlingen.

13. Verfahren zur Herstellung von Verbindungen der Formel

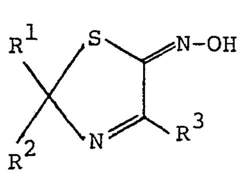

(II)

worin

$R^1$ und $R^2$ Wasserstoff, $C_{1-4}$-Alkyl oder $C_{3-6}$-Cycloalkyl, oder zusammen mit dem Kohlenstoffatom, an das sie geknüpft sind, einen 4- bis 6-gliedrigen, gesättigten Kohlenwasserstoffring und

$R^3$ Wasserstoff, $C_{1-4}$-Alkyl oder $C_{3-6}$-Cycloalkyl bedeuten, dadurch gekennzeichnet, dass man eine Verbindung der Formel

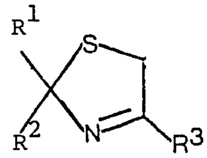

(VII)

worin $R^1$, $R^2$ und $R^3$ die oben angegebenen Bedeutungen besitzen, nitrosiert.

14. Verfahren zur Herstellung von Verbindungen der Formel II in Anspruch 13, dadurch gekennzeichnet, dass man eine Verbindung der Formel

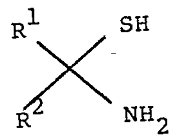

(VIII)

worin $R^1$ und $R^2$ die in Anspruch 13 angegebenen Bedeutungen besitzen, mit einer Verbindung der Formel

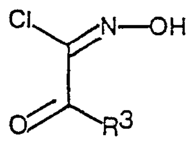

(IX)

worin $R^3$ die in Anspruch 13 angegebenen Bedeutungen besitzt, umsetzt.

15. Verfahren nach Anspruch 13 oder 14, worin $R^1$ Wasserstoff oder $C_{1-4}$-Alkyl bedeutet.

16. Verfahren nach einem der Ansprüche 13 bis 15, worin $R^2$ Wasserstoff oder $C_{1-4}$-Alkyl bedeutet.

17. Verfahren nach einem der Ansprüche 13 bis 16, worin $R^3$ Methyl bedeutet.

18. Verfahren nach Anspruch 17, dadurch gekennzeichnet, dass man 2,2,4-Trimethyl-3-thiazolin nitrosiert, um 5-Oxo-2,2,4-trimethyl-3-thiazolin-oxim herzustellen.

**Claims** (for the Contracting States : BE, CH, DE, FR, GB, IT, LI, NL)

1. Compounds of the general formula

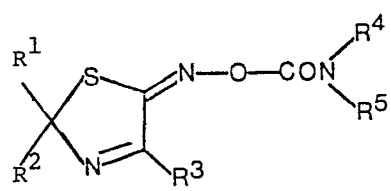

(I)

wherein

$R^1$ and $R^2$ signify hydrogen, $C_{1-4}$-alkyl or $C_{3-6}$-cycloalkyl, or together with the carbon atom to which they are attached a 4- to 6-membered, saturated hydrocarbon ring,

$R^3$ signifies hydrogen, $C_{1-4}$-alkyl or $C_{3-6}$-cycloalkyl,

$R^4$ signifies hydrogen and

$R^5$ signifies $C_{1-4}$-alkyl, $C_{2-5}$-alkenyl or $C_{3-6}$-cycloalkyl, or

$R^4$ signifies $C_{1-4}$-alkyl and

$R^5$ signifies $C_{1-4}$-alkyl, ($C_{1-4}$-alkyl)-carbonyl, tri($C_{1-4}$-alkyl)silyl or a group (a)

$$—S_m—R^6 \quad (a)$$

$R^6$ signifies $C_{1-8}$-alkyl ; $C_{1-4}$-haloalkyl ; phenyl, optionally substituted with halogen, $C_{1-4}$-alkyl and/or trifluoromethyl ; or a group (b) or (c)

$$—NR^7R^8 \quad (b)$$

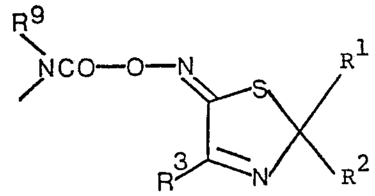

(c)

$R^7$ and $R^8$ signify $C_{1-4}$-alkyl or $C_{1-4}$-haloalkyl, or together with the nitrogen atom to which they are attached a piperidino or morpholino group,

$R^9$ signifies $C_{1-4}$-alkyl and

m signifies 1 or 2.

2. Compounds according to claim 1, wherein $R^1$ signifies hydrogen or $C_{1-4}$-alkyl.

3. Compounds according to claim 1 or 2, wherein $R^2$ signifies hydrogen or $C_{1-4}$-alkyl.

4. Compounds according to any one of claims 1 to 3, wherein $R^3$ signifies methyl.

5. Compounds according to any one of claims 1 to 4, wherein $R^4$ signifies hydrogen and $R^5$ signifies $C_{1-4}$-alkyl.

6. 5-Oxo-2,2,4-trimethyl-3-thiazoline O-(methylcarbamoyl)oxime

7. Compounds according to claim 1 as pest control agents.

8. A pest control composition, characterized in that it contains an effective amount of at least one compound of general formula I in claim 1 as well as inert carrier material.

9. A pest control composition according to claim 8, characterized in that it contains an effective amount of 5-oxo-2,2,4-trimethyl-3-thiazoline O-(methylcarbamoyl)oxime.

10. A process for the manufacture of those compounds of general formula I in claim 1 in which $R^4$ signifies hydrogen and $R^5$ signifies $C_{1-4}$-alkyl, $C_{2-5}$-alkenyl or $C_{3-6}$-cycloalkyl, characterized by reacting an oxime of the formula

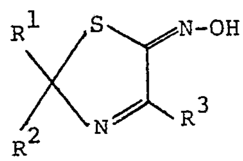

(II)

wherein $R^1$, $R^2$ and $R^3$ have the significances given in claim 1, with an isocyanate of the formula

$$R^5—N = C = O \qquad (III)$$

wherein $R^5$ has the significance given above.

11. A process for the manufacture of compounds of general formula I in claim 1, characterized by reacting an oxime of formula II in claim 10 with a carbamoyl halide of the formula

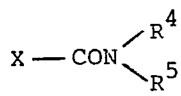

(IV)

wherein $R^4$ and $R^5$ have the significances given in claim 1 and X signifies a halogen atom.

12. A process for the manufacture of compounds of general formula I in claim 1, characterized by reacting an oxime of formula II in claim 10 with phosgene and subsequently an amine of the formula

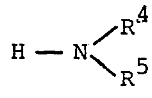

(V)

wherein $R^4$ and $R^5$ have the significances given in claim 1.

13. A process for the manufacture of those compounds of general formula I in claim 1 in which $R^4$ signifies $C_{1-4}$-alkyl and $R^5$ signifies a group (a), characterized by reacting a carbamoyl oxime of the formula

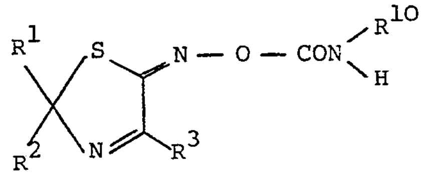

(I')

wherein $R^1$, $R^2$ and $R^3$ have the significances given in claim 1 and $R^{10}$ signifies $C_{1-4}$-alkyl, which may be obtained according to any one of claims 10 to 12, with a sulphenyl chloride of the formula

$$Cl—S_m—R^{11} \quad (VI)$$

wherein m signifies 1 or 2 and $R^{11}$ signifies one of the significances of $R^6$ given in claim 1 or chlorine.

14. The use of one of the compounds set forth in claims 1 to 7 or of a composition set forth in claim 8 for the control of pests.

15. Compounds of the general formula

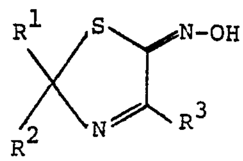

(II)

wherein

$R^1$ and $R^2$ signify hydrogen, $C_{1-4}$-alkyl or $C_{3-6}$-cycloalkyl, or together with the carbon atom to which they are attached a 4- to 6-membered, saturated hydrocarbon ring and

$R^3$ signifies hydrogen, $C_{1-4}$-alkyl or $C_{3-6}$-cycloalkyl.

16. Compounds according to claim 15, wherein $R^1$ signifies hydrogen or $C_{1-4}$-alkyl.

17. Compounds according to claim 15 or 16, wherein $R^2$ signifies hydrogen or $C_{1-4}$-alkyl.

18. Compounds according to any one of claims 15 to 17, wherein $R^3$ signifies methyl.

19. 5-Oxo-2,2,4-trimethyl-3-thiazoline oxime.

20. A process for the manufacture of compounds of formula II in claim 15, characterized by nitrosating a compound of the formula

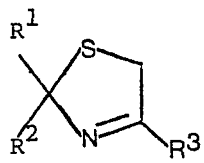

(VII)

wherein $R^1$, $R^2$ and $R^3$ have the significances given in claim 15.

21. A process for the manufacture of compounds of formula II in claim 15, characterized by reacting a compound of the formula

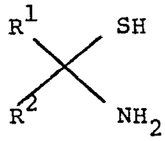

(VIII)

wherein $R^1$ and $R^2$ have the significances given in claim 15, with a compound of the formula

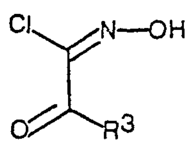

(IX)

wherein $R^3$ has the significances given in claim 15.

**Claims** (for the Contracting State AT)

1. A pest control composition, characterized in that it contains an effective amount of at least one compound of the general formula

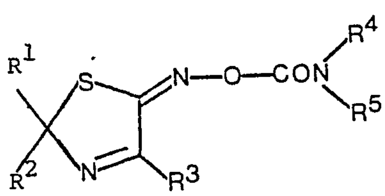

(I)

wherein

$R^1$ and $R^2$ signify hydrogen, $C_{1-4}$-alkyl or $C_{3-6}$-cycloalkyl, or together with the carbon atom to which they are attached a 4- to 6-membered, saturated hydrocarbon ring,

$R^3$ signifies hydrogen, $C_{1-4}$-alkyl or $C_{3-6}$-cycloalkyl,

$R^4$ signifies hydrogen and

$R^5$ signifies $C_{1-4}$-alkyl, $C_{2-5}$-alkenyl or $C_{3-6}$-cycloalkyl, or

$R^4$ signifies $C_{1-4}$-alkyl and

$R^5$ signifies $C_{1-4}$-alkyl, ($C_{1-4}$-alkyl)-carbonyl, tri($C_{1-4}$-alkyl)silyl or a group (a)

$$—S_m—R^6 \qquad (a)$$

$R^6$ signifies $C_{1-8}$-alkyl ; $C_{1-4}$-haloalkyl ; phenyl, optionally substituted with halogen, $C_{1-4}$-alkyl and/or trifluoromethyl ; or a group (b) or (c)

$$—NR^7R^8 \qquad (b)$$

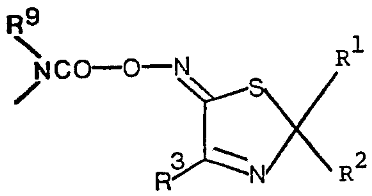

(c)

$R^7$ and $R^8$ signify $C_{1-4}$-alkyl or $C_{1-4}$-haloalkyl, or together with the nitrogen atom to which they are attached a piperidino or morpholino group,

$R^9$ signifies $C_{1-4}$-alkyl and

m signifies 1 or 2, as well as inert carrier material.

2. A pest control composition according to claim 1, wherein $R^1$ signifies hydrogen or $C_{1-4}$-alkyl.

3. A pest control composition according to claim 1 or 2, wherein $R^2$ signifies hydrogen or $C_{1-4}$-alkyl.

4. A pest control composition according to any one of claims 1 to 3, wherein $R^3$ signifies methyl.

5. A pest control composition according to any one of claims 1 to 4, wherein $R^4$ signifies hydrogen and $R^5$ signifies $C_{1-4}$-alkyl.

6. A pest control composition according to claim 1, characterized in that it contains an effective amount of 5-oxo-2,2,4-trimethyl-3-thiazoline O-(methylcarbamoyl) oxime as well as inert carrier material.

7. A process for the manufacture of those compounds of general formula I in claim 1 in which $R^4$ signifies hydrogen and $R^5$ signifies $C_{1-4}$-alkyl, $C_{2-5}$-alkenyl or $C_{3-6}$-cycloalkyl, characterized by reacting an oxime of the formula wherein $R^1$, $R^2$ and $R^3$ have the significances given in claim 1, with an isocyanate of the formula

$$R^5—N = C = O \qquad (III)$$

wherein $R^5$ has the significance given above.

8. A process for the manufacture of compounds of general formula I in claim 1, characterized by reacting an oxime of formula II in claim 7 with a carbamoyl halide of the formula

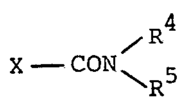

(IV)

wherein $R^4$ and $R^5$ have the significances given in claim 1 and X signifies a halogen atom.

9. A process for the manufacture of compounds of general formula I in claim 1, characterized by reacting an oxime of formula II in claim 7 with phosgene and subsequently an amine of the formula

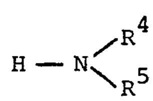

(V)

wherein $R^4$ and $R^5$ have the significances given in claim 1.

10. A process for the manufacture of those compounds of general formula I in claim 1 in which $R^4$ signifies $C_{1-4}$-alkyl and $R^5$ signifies a group (a), characterized by reacting a carbamoyl oxime of the formula

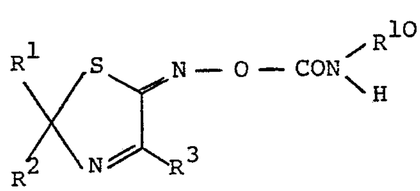

I'

wherein $R^1$, $R^2$ and $R^3$ have the significances given in claim 1 and $R^{10}$ signifies $C_{1-4}$-alkyl, which may be obtained according to any one of claims 7 to 9, with a sulphenyl chloride of the formula

$$Cl—S_m—R^{11} \qquad (VI)$$

wherein m signifies 1 or 2 and $R^{11}$ signifies one of the significances of $R^6$ given in claim 1 or chlorine.

11. A process for the manufacture of a pest control composition, characterized by mixing at least one of the compounds set forth in claims 1 to 6 with an inert carrier material.

12. The use of one of the compounds or compositions set forth in claims 1 to 6 for the control of pests.

13. A process for the manufacture of compounds of the formula

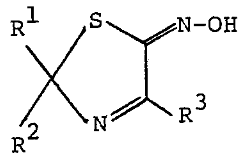

(II)

wherein

$R^1$ and $R^2$ signify hydrogen, $C_{1-4}$-alkyl or $C_{3-6}$-cycloalkyl, or together with the carbon atom to which they are attached a 4- to 6-membered, saturated hydrocarbon ring and

$R^3$ signifies hydrogen, $C_{1-4}$-alkyl or $C_{3-6}$-cycloalkyl, characterized by nitrosating a compound of the formula

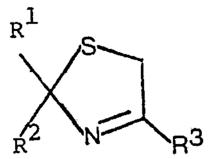

(VII)

wherein $R^1$, $R^2$ and $R^3$ have the significances given above.

14. A process for the manufacture of compounds of formula II in claim 13, characterized by reacting a compound of the formula

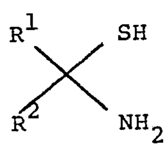

(VIII)

wherein $R^1$ and $R^2$ have the significances given in claim 13, with a compound of the formula

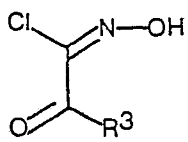

(IX)

wherein $R^3$ has the significances given in claim 13.

15. A process according to claim 13 or 14, wherein $R^1$ signifies hydrogen or $C_{1-4}$-alkyl.

16. A process according to any one of claims 13 to 15, wherein $R^2$ signifies hydrogen or $C_{1-4}$-alkyl.

17. A process according to any one of claims 13 to 16, wherein $R^3$ signifies methyl.

18. A process according to claim 17, characterized by nitrosating 2,2,4-trimethyl-3-thiazoline in order to prepare 5-oxo-2,2,4-trimethyl-3-thiazoline oxime.

**Revendications** (pour les Etats contractants : BE, CH, DE, FR, GB, IT, LI, NL)

1. Composés de formule générale

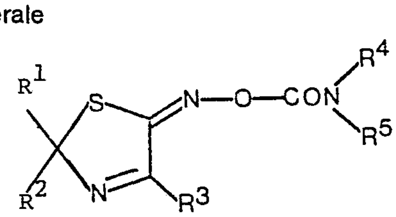

(I)

$R^1$ et $R^2$ représentent l'hydrogène, des groupes alkyles en $C_1$-$C_4$ ou cycloalkyles en $C_3$-$C_6$ ou bien forment avec l'atome de carbone sur lequel ils sont fixés, un cycle hydrocarboné saturé de 4 à 6 chaînons,

$R^3$ représente l'hydrogène, un groupe alkyle en $C_1$-$C_4$ ou cycloalkyle en $C_3$-$C_6$,
$R^4$ représente l'hydrogène, et
$R^5$ représente un groupe alkyle en $C_1$-$C_4$, alcényle en $C_2$-$C_5$ ou cycloalkyle en $C_3$-$C_6$, ou bien
$R^4$ représente un groupe alkyle en $C_1$-$C_4$ et
$R^5$ représente un groupe alkyle en $C_1$-$C_4$, (alkyle en $C_1-C_4$)-carbonyle, tri-(alkyle en $C_1$-$C_4$)-silyle ou un groupe (a)

$$—S_m—R^6 \qquad (a)$$

$R^6$ représente un groupe alkyle en $C_1$-$C_8$ ; halogénoalkyle en $C_1$-$C_4$ ; phényle éventuellement substitué par un halogène, un groupe alkyle en $C_1$-$C_4$ et/ou un groupe trifluorométhyle ; ou un groupe (b) ou (c)

$$—NR^7R^8 \qquad (b)$$

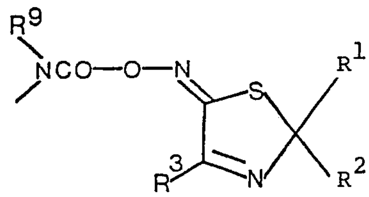

(c)

$R^7$ et $R^8$ représentent des groupes alkyles en $C_1$-$C_4$ en halogénoalkyles en $C_1$-$C_4$, ou forment ensemble et avec l'atome d'azote sur lequel ils sont fixés un groupe pipéridino ou morpholino,
$R^9$ représente un groupe alkyle en $C_1$-$C_4$ et
m est égal à 1 ou 2.

2. Composés selon la revendication 1 dans lesquels $R^1$ représente l'hydrogène ou un groupe alkyle en $C_1$-$C_4$.

3. Composés selon la revendication 1 ou 2, dans lesquels $R^2$ représente l'hydrogène ou un groupe alkyle en $C_1$-$C_4$.

4. Composés selon l'une des revendications 1 à 3, dans lesquels $R^3$ représente un groupe méthyle.

5. Composés selon l'une des revendications 1 à 4, dans lesquels $R^4$ représente l'hydrogène et $R^5$ un groupe alkyle en $C_1$-$C_4$.

6. La 5-oxo-2,2,4-triméthyl-3-thiazoline-O-(méthylcarbamoyl)-oxime.

7. Composés selon la revendication 1, en tant que pesticides.

8. Produit pesticide caractérisé en ce qu'il contient une quantité efficace d'au moins un composé de formule générale I de la revendication 1 et un véhicule inerte.

9. Produit pesticide selon la revendication 8, caractérisé en ce qu'il contient une quantité efficace de 5-oxo-2,2,4-triméthyl-3-thiazoline-O-(méthyl-carbamoyl)-oxime.

10. Procédé de préparation des composés de formule générale I de la revendication 1 dans laquelle $R^4$ représente l'hydrogène et $R^5$ un groupe alkyle en $C_1$-$C_4$, alcényle en $C_2$-$C_5$ ou cycloalkyle en $C_3$-$C_6$, caractérisé en ce que l'on fait réagir une oxime de formule

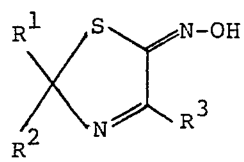

(II)

dans laquelle $R^1$, $R^2$ et $R^3$ ont les significations indiquées dans la revendication 1, avec un isocyanate de formule

$$R^5—N = C = O \qquad (III)$$

dans laquelle $R^5$ a la signification indiquée ci-dessus.

11. Procédé de préparation des composés de formule générale I de la revendication 1, caractérisé en ce que l'on fait réagir une oxime de formule II de la revendication 10 avec un halogénure de carbamoyle de formule

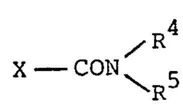

(IV)

dans laquelle $R^4$ et $R^5$ ont les significations indiquées dans la revendication 1 et X représente un atome d'halogène.

12. Procédé de préparation des composés de formule générale I de la revendication 1, caractérisé en ce que l'on fait réagir une oxime de formule II de la revendication 10 avec le phosgène puis une amine de formule

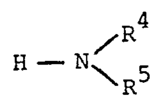

(V)

dans laquelle $R^4$ et $R^5$ ont les significations indiquées dans la revendication 1.

13. Procédé de préparation des composés de formule générale I de la revendication 1 dans laquelle $R^4$ représente un groupe alkyle en $C_1$-$C_4$ et $R^5$ un groupe (a), caractérisé en ce que l'on fait réagir une carbamoyl-oxime éventuellement obtenue selon l'une des revendications 10 à 12 et répondant à la formule

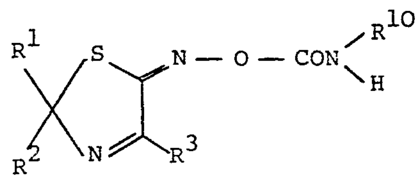

(I')

dans laquelle $R^1$, $R^2$ et $R^3$ ont les significations indiquées dans la revendication 1 et $R^{10}$ représente un groupe alkyle en $C_1$-$C_4$, avec un chlorure de sulfényle de formule

$$Cl—S_m—R^{11} \qquad (VI)$$

dans laquelle m est égal à 1 ou 2 et $R^{11}$ a l'une des significations indiquées pour $R^6$ dans la revendication 1 ou représente le chlore.

14. Utilisation de l'un des composés mentionnés dans les revendications 1 à 7 ou d'un produit mentionné dans la revendication 8 pour la lutte contre les parasites.

15. Composés de formule générale

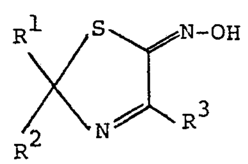

(II)

dans laquelle

$R^1$ et $R^2$ représentent l'hydrogène, des groupes alkyles en $C_1$-$C_4$ ou cycloalkyles en $C_3$-$C_6$, ou bien forment ensemble et avec l'atome de carbone sur lequel ils sont fixés un cycle hydrocarboné saturé de 4 à 6 chaînons, et

$R^3$ représente l'hydrogène, un groupe alkyle en $C_1$-$C_4$ ou cycloalkyle en $C_3$-$C_6$.

16. Composés selon la revendication 15, dans lesquels $R^1$ représente l'hydrogène ou un groupe alkyle en $C_1$-$C_4$.

17. Composés selon la revendication 15 ou 16, dans lesquels $R^2$ représente l'hydrogène ou un groupe alkyle en $C_1$-$C_4$.

18. Composés selon l'une des revendications 15 à 17, dans lesquels $R^3$ représente un groupe méthyle.

19. La 5-oxo-2,2,4-triméthyl-3-thiazoline-oxime.

20. Procédé de préparation des composés de formule II de la revendication 15, caractérisé en ce que l'on nitrose un composé de formule

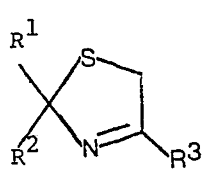

(VII)

dans laquelle $R^1$, $R^2$ et $R^3$ ont la signification indiquée dans la revendication 15.

21. Procédé de préparation des composés de formule II de la revendication 15, caractérisé en ce que l'on fait réagir un composé de formule

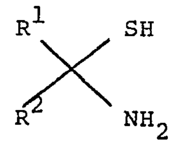

(VIII)

dans laquelle $R^1$ et $R^2$ ont les significations indiquées dans la revendication 15, avec un composé de formule

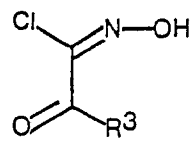

(IX)

dans laquelle $R^3$ a les significations indiquées dans la revendication 15.

**Revendications** (pour L'Etat contractant AT)

1. Produit pesticide caractérisé en ce qu'il contient une quantité efficace d'au moins un composé de formule générale

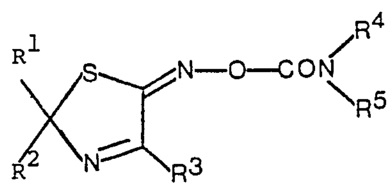

(I)

dans laquelle

$R^1$ et $R^2$ représentent l'hydrogène, des groupes alkyles en $C_1$-$C_4$, cycloalkyles en $C_3$-$C_6$, ou bien forment ensemble et avec l'atome de carbone sur lequel ils sont fixés un cycle hydrocarboné saturé de 4 à 6 chaînons,

$R^3$ représente l'hydrogène, un groupe alkyle en $C_1$-$C_4$ ou cycloalkyle en $C_3$-$C_6$,

$R^4$ représente l'hydrogène, et

$R^5$ représente un groupe alkyle en $C_1$-$C_4$, alcényle en $C_2$-$C_5$ ou cycloalkyle en $C_3$-$C_6$, ou bien

$R^4$ représente un groupe alkyle en $C_1$-$C_4$ et

$R^5$ représente un groupe alkyle en $C_1$-$C_4$, (alkyle en $C_1-C_4$)-carbonyle, tri-(alkyle en $C_1$-$C_4$)-silyle ou un groupe (a)

$$—S_m—R^6 \qquad (a)$$

$R^6$ représente un groupe alkyle en $C_1$-$C_8$ ; halogénoalkyle en $C_1$-$C_4$ ; phényle éventuellement substitué par un halogène, un groupe alkyle en $C_1$-$C_4$ et/ou trifluorométhyle ; ou bien un groupe (b) ou (c)

$$—NR^7R^8 \qquad (b)$$

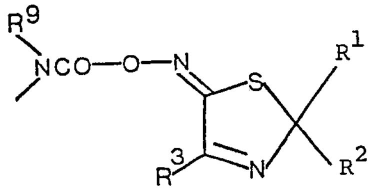

(c)

$R^7$ et $R^8$ représentent des groupes alkyles en $C_1$-$C_4$ ou halogénoalkyles en $C_1$-$C_4$, ou bien forment ensemble et avec l'atome d'azote sur lequel ils sont fixés un groupe pipéridino ou morpholino,

$R^9$ représente un groupe alkyle en $C_1$-$C_4$ et

m est égal à 1 ou 2, et un véhicule inerte.

2. Produit pesticide selon la revendication 1 dans lequel $R^1$ représente l'hydrogène ou un groupe alkyle en $C_1$-$C_4$.

3. Produit pesticide selon la revendication 1 ou 2, dans lequel $R^2$ représente l'hydrogène ou un groupe alkyle en $C_1$-$C_4$.

4. Produit pesticide selon l'une des revendications 1 à 3, dans lequel $R^3$ représente un groupe méthyle.

5. Produit pesticide selon l'une des revendications 1 à 4, dans lequel $R^4$ représente l'hydrogène et $R^5$ un groupe alkyle en $C_1$-$C_4$.

6. Produit pesticide selon la revendication 1, caractérisé en ce qu'il contient une quantité efficace de 5-oxo-2,2,4-triméthyl-3-thiazoline-O-(méthyl-carbamoyl)-oxime et un véhicule inerte.

7. Procédé de préparation des composés de formule générale I de la revendication 1 dans laquelle $R^4$ représente l'hydrogène et $R^5$ un groupe alkyle en $C_1$-$C_4$, alcényle en $C_2$-$C_5$ ou cycloalkyle en $C_3$-$C_6$, caractérisé en ce que l'on fait réagir une oxime de formule

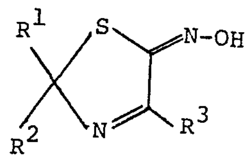

(II)

dans laquelle $R^1$, $R^2$ et $R^3$ ont les significations indiquées dans la revendication 1, avec un isocyanate de formule

$$R^5—N = C = O \qquad \text{(III)}$$

dans laquelle $R^5$ a la signification indiquée ci-dessus.

8. Procédé de préparation des composés de formule générale I de la revendication 1, caractérisé en ce que l'on fait réagir une oxime de formule II de la revendication 7 avec un halogénure de carbamoyle de formule

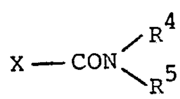

(IV)

dans laquelle $R^4$ et $R^5$ ont les significations indiquées dans la revendication 1 et X représente un atome d'halogène.

9. Procédé de préparation des composés de formule générale I de la revendication 1, caractérisé en ce que l'on fait réagir une oxime de formule II de la revendication 7 avec le phosgène puis avec une amine de formule

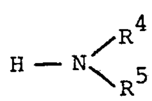

(V)

dans laquelle $R^4$ et $R^5$ ont les significations indiquées dans la revendication 1.

10. Procédé de préparation des composés de formule générale I de la revendication 1 dans laquelle $R^4$ représente un groupe alkyle en $C_1$-$C_4$ et $R^5$ un groupe (a), caractérisé en ce que l'on fait réagir une carbamoyloxime éventuellement obtenue selon l'une des revendications 7 à 9 et répondant à la formule

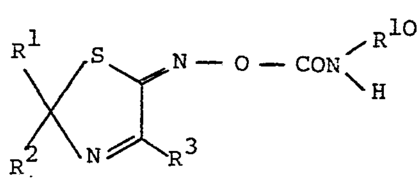

(I')

dans laquelle $R^1$, $R^2$ et $R^3$ ont les significations indiquées dans la revendication 1 et $R^{10}$ représente un groupe alkyle en $C_1$-$C_4$ avec un chlorure de sulfényle de formule

$$Cl—S_m—R^{11} \qquad \text{(VI)}$$

dans laquelle m est égal à 1 ou 2 et $R^{11}$ a l'une des significations indiquées pour $R^6$ dans la revendication 1 ou représente le chlore.

11. Procédé de préparation d'un produit pesticide, caractérisé en ce que l'on mélange au moins un des composés mentionnés dans les revendications 1 à 6 avec un véhicule inerte.

12. Utilisation de l'un des composés ou produits mentionnés dans les revendications 1 à 6 dans la lutte contre les pesticides.

13. Procédé de préparation des composés de formule

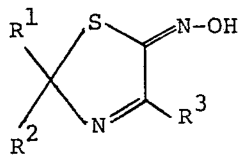

(II)

dans laquelle

$R^1$ et $R^2$ représentent l'hydrogène, des groupes alkyles en $C_1$-$C_4$ ou cycloalkyles en $C_3$-$C_6$ ou bien forment ensemble et avec l'atome de carbone sur lequel ils sont fixés un cycle hydrocarboné saturé de 4 à 6 chaînons et

$R^3$ représente l'hydrogène, un groupe alkyle en $C_1$-$C_4$ ou cycloalkyle en $C_3$-$C_6$, caractérisé en ce que l'on nitrose un composé de formule

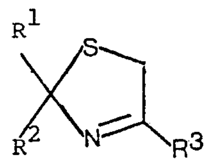

(VII)

dans laquelle $R^1$, $R^2$ et $R^3$ ont les significations indiquées ci-dessus.

14. Procédé de préparation des composés de formule II de la revendication 13, caractérisé en ce que l'on fait réagir un composé de formule

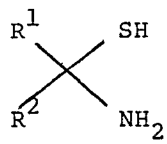

(VIII)

dans laquelle $R^1$ et $R^2$ ont les significations indiquées dans la revendication 13, avec un composé de formule

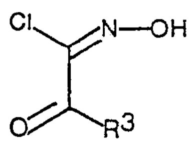

(IX)

dans laquelle $R^3$ a les significations indiquées dans la revendication 13.

15. Procédé selon la revendication 13 ou 14, dans lequel $R^1$ représente l'hydrogène ou un groupe alkyle en $C_1$-$C_4$.

16. Procédé selon l'une des revendications 13 à 15, dans lequel $R^2$ représente l'hydrogène ou un groupe alkyle en $C_1$-$C_4$.

17. Procédé selon l'une des revendications 13 à 16, dans lequel $R^3$ représente un groupe méthyle.

18. Procédé selon la revendication 17, caractérisé en ce que, pour la préparation de la 5-oxo-2,2,4-triméthyl-3-thiazoline-oxime, on nitrose la 2,2,4-triméthyl-3-thiazoline.